# EUROPÄISCHE PATENTSCHRIFT

(11) **EP 3 587 554 B1**
(45) Veröffentlichungstag und Bekanntmachung des Hinweises auf die Patenterteilung: **23.12.2020**
(21) Anmeldenummer: 19182290.7
(22) Anmeldetag: 25.06.2019
(51) Int. Cl.: C12M 1/34, C12M 1/00, C12M 1/36, B01J 19/00

(54) **VERFAHREN ZUR BESCHLEUNIGTEN BESTIMMUNG DER KONZENTRATION LEBENDER THERMOPHILER BAKTERIEN IN WASSERFÜHRENDEN ANLAGEN**
METHOD FOR ACCELERATED DETERMINATION OF THE CONCENTRATION OF LIVING THERMOPHILIC BACTERIA IN WATER-BEARING INSTALLATIONS
PROCÉDÉ DE DÉTERMINATION ACCÉLÉRÉE DE LA CONCENTRATION DE BACTÉRIES THERMOPHILES VIVANTES DANS LES INSTALLATIONS D'ACHEMINEMENT D'EAU

(30) Priorität: 26.06.2018 DE 102018115381
(43) Veröffentlichungstag der Anmeldung: 01.01.2020
(73) Patentinhaber: Inwatec GmbH & Co. KG, 50127 Bergheim (DE)
(72) Erfinder: Ohme, Holger, 50858 Köln (DE)
(74) Vertreter: Geskes, Christoph

(56) Entgegenhaltungen:
- WO-A1-2009/121726
- WO-A1-2011/151793
- US-B2- 7 695 957
- C. MOLINER ET AL: "Rapid identification of Legionella species by mass spectrometry", JOURNAL OF MEDICAL MICROBIOLOGY, Bd. 59, Nr. 3, 15. November 2009 (2009-11-15), Seiten 273-284, XP055610062, ISSN: 0022-2615, DOI: 10.1099/jmm.0.014100-0
- LOURENÃ O N D ET AL: "Bioreactor monitoring with spectroscopy and chemometrics: a review", ANALYTICAL AND BIOANALYTICAL CHEMISTRY, SPRINGER, BERLIN, DE, Bd. 404, Nr. 4, 30. Mai 2012 (2012-05-30), Seiten 1211-1237, XP035098919, ISSN: 1618-2650, DOI: 10.1007/S00216-012-6073-9

## Beschreibung

Die vorliegende Erfindung betrifft ein Verfahren zur beschleunigten Bestimmung der Konzentration lebender thermophiler Bakterien in wasserführenden Anlagen, insbesondere in Kühlwasserkreisläufen, als auch eine Vorrichtung zur Durchführung des erfindungsgemäßen Verfahrens.

Wasserführende Anlagen umfassen im Sinne der vorliegenden Erfindung beispielhaft Kühlwasserkreisläufe, die mit Wasser betrieben werden, wie diese beispielsweise bei Verdunstungskühlanlagen vorhanden sind. Wasserführende Anlagen finden sich jedoch auch beispielweise bei Wasserversorgungsunternehmen, Betreibern von großen Waschkauen oder aber in Schwimmbädern beispielhaft wieder. Wasserführende Anlagen weisen oftmals Verunreinigungen auf. Diese betreffen nicht nur anorganische Ablagerungen wie Kalk, sondern auch organische Ablagerungen, welche oftmals in Form von Biofilmen sich in wasserführenden Anlagen wiederfinden. Besonders kritisch ist eine Verunreinigung von wasserführenden Anlagen dann, wenn sich in diesen pathogene Mikroorganismen verbreiten bzw. vermehren. Besonders hervorzuheben ist hier das thermophile Bakterium Legionella spp. (Legionella species pluralis). Daher wurden auch von den Gesetzgebern Vorgaben für den hygienischen Betrieb wasserführender Anlagen erlassen, in der Bundesrepublik Deutschland beispielweise im Rahmen der 42. Verordnung zum Bundesemissionsschutzgesetz.

Betreiber von wasserführenden Anlagen überwachen diese auf Verunreinigungen insbesondere von pathogenen Mikroorganismen, oder lassen eine Überwachung durch Drittunternehmen durchführen. Um eine Verunreinigung mit pathogenen Mikroorganismen festzustellen, werden in akkreditierten Messverfahren letztendlich Kolonien der pathogenen Mikroorganismen auf Nährboden erzeugt und hieraus der Befall an sich als auch die Stärke des Befalls ermittelt. Derartige, auf Selektivnährböden nachgewiesene koloniebildende Einheiten benötigen eine Untersuchungsdauer von üblicherweise 10 bis 12 Tagen bei langsamwachsenden Bakterien wie Legionella spp., ansonsten überlicherweise mehrere Tage. Der Betreiber einer wasserführenden Anlage muss daher zumindest diesen Zeitraum abwarten und wird erst nach Befundmitteilung tätig werden können in dem Sinne, dass er gegen pathogene Mikroorganismen vorgeht. In diesem Zeitraum haben sich damit schon pathogene Mikroorganismen in der wasserführenden Anlage weiter vermehrt und verbreitet.

WO 2011/151 793 A1 offenbart ein Verfahren zum Nachweis und zur Zählung lebensfähiger Mikroorganismen in einer Probe, bei der der Verdacht besteht, dass sie diese Mikroorganismen enthält: (1) Inkontaktbringen der Mikroorganismen der Probe mit Reparaturverbindungen und einem Wachstumsmedium, und (2) Inkubieren des Produkts aus Schritt (1), und (3) Nachweisen und Zählen der Mikroorganismen, wobei die Mikroorganismen von der Art Legionella pneumophila sind, und wobei die Reparaturverbindungen (a) Serin; (b) Threonin; (c) eine Verbindung, die Calciumionen in einer Dosis von 10⁻⁶ bis 10⁻² mM enthält;(d)eine Verbindung, die Magnesiumionen in einer Dosis von 10⁻⁶ bis 10⁻² mM enthält; (e) eine Verbindung, die Kaliumionen enthält; (f) Glutaminsäure oder ein Salz davon; und (g) Brenztraubensäure oder ein Salz davon, umfassen. Es ist auch ein Kit zum Nachweis und zur Auszählung lebensfähiger Mikroorganismen der Art Legionella pneumophila in einer Probe, die vermutlich diese Mikroorganismen enthält, offenbart.

US 7 695 957 B2 offenbart einen Bioreaktor, umfassend einen Behälter, der eine Zellkultur enthalten kann, eine erste Zufuhrleitung für den Zustrom eines Kulturmediums in das Innere des Behälters und eine zweite Ausgangsleitung für den Abfluss des Kulturmediums aus dem Behälter, wobei die erste Zufuhrleitung mit einem spiralförmigen Kapillarrohr verbunden ist, wobei das spiralförmige Kapillarrohr eine solche Form hat, dass, wenn das Medium innerhalb der ersten Zufuhrleitung zum Fließen gebracht und aus der zweiten Ausgangsleitung zum Ausfließen gebracht wird, der hydrostatische Schub und die hydrodynamischen Kräfte einen Zustand simulierter reduzierter Schwerkraft in Bezug auf die Zellen im Inneren des Behälters erzeugen.

Um diesen Nachteil auszugleichen, d. h. dem Betreiber einer wasserführenden Anlage zu ermöglichen, innerhalb kurzer Zeiträume, idealerweise Zeiträumen von 1 bis 2 Tagen, Informationen über einen Befall mit pathogenen Mikroorganismen zu erhalten, sind Schnelltestmethoden entwickelt worden. Diesen liegen immunologische Nachweisreaktionen oder Analysen des genetischen Materiales unter Verwendung der PCR-Methode (PCR = Polymer Chain Reaction) zugrunde. Diese beiden Nachweismethoden liefern jedoch nur eine generelle Aussage über das Vorhandensein der pathogenen Mikroorganismen an sich, keine verlässliche lebend/tot Differenzierung der Mikroorganismen, und auch keine Aussage über eine Konzentration der Mikroorganismen. Auch ist mittels dieser Schnelltestmethoden keine Korrelation mit der vorstehend beschriebenen Methode der Koloniebildung von pathogenen Mikroorganismen auf Nährboden als akkreditiertes Messverfahren möglich. Im Unterschied zu der koloniebildenden Methode unter Einsatz von Nährböden, welche lebende und damit in aller Regel auch aktive pathogene Mikroorganismen nachweist, werden bei den beiden genannten Schnelltestmethoden keine Nachweise lebender pathogener Mikroorganismen erbracht.

Um diesen Nachteil einer mangelnden Korrelation im Rahmen eines Schnelltests zu beheben, kann man andenken, bei immunologischen Nachweismethoden zunächst lebende von toten pathogenen Mikroorganismen zu trennen. Eine derartige Trennung ist jedoch sehr aufwändig und erfordert eine Vielzahl von zusätzlichen Arbeitsschritten, die zeit- und kostenintensiv sind. Eine Automatisierung ist kaum denkbar. Auch sind solche Methoden je nach Art der wasserführenden Anlage auch nur beschränkt einsetzbar. Beispielweise weisen Kühlwasserkreisläufe oftmals Öle auf oder sind sonst wie feststoffbelastet, so dass die Trennung von lebenden und toten pathogenen Mikroorganismen kaum möglich ist. Zudem sind die bekannten Schnelltestmethoden und deren Verfahren entweder gar nicht bei derartigen starkbelasteten industriellen wasserführenden Anlagen einsetzbar oder aber liefern nicht belastbare Ergebnisse.

Aus dem Stand der Technik sind als Methode zur schnellen Erfassung einer Lebendzellzahl solche bekannt, die auf einer Impedanzmessung basieren. Hierdurch ist dann zwar unter Umständen auch eine Korrelation mit koloniebildenden Einheiten unter Einsatz eines Nährbodens, wie vorstehend beschrieben, möglich, jedoch ist eine zuverlässige Differenzierung zwischen allgemeinen Keimen und thermophilen Bakterien und insbesondere pathogenen thermophilen Bakterien wie Legionella spp. mit derartigen Verfahren nicht möglich.

Die vorliegende Erfindung stellt sich daher die Aufgabe, ein Verfahren und eine Vorrichtung zur beschleunigten Bestimmung der Konzentration lebender thermophiler Bakterien in wasserführenden Anlagen, insbesondere pathogener thermophiler Bakterien, zur Verfügung zu stellen, wobei das Verfahren schnell, d. h. innerhalb eines Tages, insbesondere innerhalb nur mehrerer Stunden, durchführbar ist und mittels des Verfahrens und der Vorrichtung eine weitgehende Automatisierung ermöglicht ist. Zudem ist es Aufgabe der vorliegenden Erfindung, ein Verfahren und eine Vorrichtung zur beschleunigten Bestimmung der Konzentration lebender thermophiler Bakterien, insbesondere pathogener thermophiler Bakterien wie Legionella spp., in wasserführenden Anlagen zur Verfügung zu stellen, mittels welchem eine Korrelation mit akkreditierten Verfahren unter Einsatz von Selektivnährböden und Auswertung koloniebildender Einheiten der untersuchten Bakterien ermöglicht ist.

Diese Aufgabe wird erfindungsgemäß gelöst durch ein Verfahren zur beschleunigten Bestimmung der Konzentration lebender thermophiler Bakterien in wasserführenden Anlagen, insbesondere in Kühlwasserkreisläufen, bei welchem ein Bioreaktor mit einer wasserführenden Zuführleitung mit mindestens einer ersten Regeleinheit für eine wässrige Messlösung und einer Abführleitung mit mindestens einer zweiten Regeleinheit verbunden ist, umfassend die Schritte
a) Beschicken des Bioreaktors mit der wässrigen Messlösung;
b) physikalische und chemische Deaktivierung von in der Messlösung enthaltener Begleitflora, welche nicht den zu bestimmenden thermophilen Bakterien, wie beispielsweise Legionella spp., entspricht, Bioziden und/oder von Enzymen bei einer erhöhten Temperatur T1 über einen Zeitraum von mindestens etwa 1 min, wobei die physikalische und/oder chemische Deaktivierung derart erfolgt, dass die zu bestimmenden thermophilen Bakterien nicht deaktiviert werden;
c) Absenken der Temperatur der Messlösung auf eine Temperatur T2 auf einen Bereich von etwa 38°C bis etwa 45 °C;
d) Zudosieren mindestens einer Indikatorlösung;
e) nach Durchführen der Schritte b) bis d) spektrometrisches Bestimmen mindestens zweier zu unterschiedlichen Zeitpunkten ermittelter Messwerte W1 und W2 der Messlösung durch mikrobiologischen Umsatz einer Indikatorlösung umfassend mindestens ein Indikatormittel; und
f) Auswerten der bestimmten mindestens zwei Messwerte W1 und W2 und Ermitteln einer Konzentration der lebenden thermophilen Bakterien in der Messlösung.

Mittels des erfindungsgemäßen Verfahrens ist es vorteilhafterweise möglich, auf lebende thermophile Bakterien abzustellen, da der Umsatz des Indikatormittels letztendlich durch mikrobiologische Aktivität der thermophilen Bakterien erfolgt. Durch die spezifische Verfahrensführung werden sonstige Bakterien so gut wie vollständig deaktiviert, so dass die spektrometrische Bestimmung durchgeführt wird an einer Messlösung, in welcher noch lebende thermophile Bakterien, insbesondere lebende pathogene thermophile Bakterien wie Legionella spp., vorhanden sind.

Das erfindungsgemäße Verfahren kann dabei weitgehend automatisiert durchgeführt werden. Eine erfindungsgemäße Vorrichtung, die das erfindungsgemäße Verfahren nutzt und die weiter unten beschrieben ist, kann dabei in eine bestehende wasserführende Anlage, beispielsweise einen Kühlwasserkreislauf, eingefügt werden über übliche, beispielsweise Flanschverbindungen. Die erste Regeleinheit und die zweite Regeleinheit können dabei zeitgesteuert automatisch geöffnet und geschlossen werden, so dass hierdurch der Bioreaktor mit der Messlösung, beispielsweise aus einem Kühlwasserkreislauf entnommenes Nutzwasser, beschickt wird. Die erste Regeleinheit und die zweite Regeleinheit sind üblicherweise als übliche Absperrogane, insbesondere in Form von Ventilen, ausgebildet und können beispielsweise als Magnetventile ausgebildet sein. Aber auch jede andere Art der Ausbildung der Ventile ist möglich, beispielsweise können auch Membranventile eingesetzt werden. Soweit keine Automatisierung vorgesehen ist, können diese auch von Hand geregelt werden. Zur Füllung des Bioreaktors wird beispielsweise die erste Regeleinheit geöffnet und nach einem gewissen Zeitraum des Durchflusses durch den Bioreaktor die zweite Regeleinheit, die in der Abführleitung angeordnet ist, geschlossen und anschließend die erste Regeleinheit, die in der wasserführenden Zuführleitung angeordnet ist, ebenfalls geschlossen, sobald ausreichend Messlösung im Bioreaktor vorliegt. Die Messung im Bioreaktor kann dann in einer nicht umlaufenden oder den Bioreaktor durchfließenden Messlösung vorgenommen werden. Der Bioreaktor kann hierfür mit einem Füllstandsensor versehen sein, der eine hinreichende Befüllung sicherstellt. Üblicherweise ist die wasserführende Zuführleitung seitlich am Bioreaktor angeordnet. Die Abführleitung ist bevorzugt im Boden des Bioreaktors, oder aber nahe dem Boden an einer Seite desselben, angeordnet. Der Bioreaktor umfasst beispielsweise ein zylinderförmiges Gehäuse, wobei das Gehäuse jedoch auch jede andere Art der Ausbildung aufweisen kann.

Nachdem gemäß Schritt a), wie insbesondere vorstehend beschrieben, der Bioreaktor mit der wässrigen Messlösung beschickt ist, werden zur Erzielung einer hohen Genauigkeit bei der Bestimmung der Konzentration lebender thermophiler Bakterien mit dem erfindungsgemäßen Verfahren eine in der Messlösung enthaltene Begleitflora, Biozide und/oder Enzyme physikalisch und/oder chemisch in einem Schritt b) deaktiviert. Die physikalische und/oder chemische Deaktivierung erfolgt erfindungsgemäß derart, dass hierdurch die zu bestimmenden thermophilen Bakterien nicht deaktiviert werden. Ebenfalls kann im Schritt b) gleichzeitig oder nachfolgend zur einer physikalischen und einer ersten chemischen Deaktivierung, die insbesondere pH-Wert-basiert durchgeführt wird, eine physikalische und chemische Deaktivierung von möglicherweise in der Messlösung enthaltenen Bioziden und/oder Enzymen, die ein Messergebnis ebenfalls verfälschen können, erfolgen. Oxidierende und/oder nichtoxidierende Biozide, die in üblicherweise geringen Mengen in einer Messlösung enthalten sein können, würden durch ein mögliches Abtöten der zu bestimmenden thermophilen Bakterien ein Messergebnis verfälschen, Enzyme würden einen Umsatz des von der Indikatorlösung umfassten mindestens einen Indikatormittels erzeugen, so dass hierdurch das Messergebnis verfälscht werden würde. Die physikalische Deaktivierung erfolgt bevorzugt bei einer erhöhten Temperatur T1 über einen Zeitraum von mindestens 1 min, weiter bevorzugt über einen Zeitraum von mindestens 5 min, weiter bevorzugt über einen Zeitraum in einem Bereich von etwa 5 min bis etwa 120 min, weiter bevorzugt über einen Zeitraum in einem Bereich von etwa 10 min bis etwa 150 min, noch weiter bevorzugt über einen Zeitraum von etwa 20 min bis etwa 120 min, und noch weiter bevorzugt über einen Zeitraum von etwa 40 min bis etwa 100 min. Auch eine chemische Deaktivierung kann bei solchen erhöhten Temperaturen durchgeführt werden. Erfindungsgemäß erfolgt die chemische Deaktivierung durch eine Einstellung des pH-Wertes, bevorzugt auf einen Wert von unter etwa 6,5, weiter bevorzugt auf einen Wert in einem Bereich von etwa 5,5 bis etwa 6,5. Besonders bevorzugt wird zur Einstellung des pH-Wertes im Rahmen der chemischen Deaktivierung mindestens ein pH-Wert-Regulierungsmittel ausgewählt, welches ausgewählt ist aus einer Gruppe umfassend mindestens eine organische Säure, bevorzugt ausgewählt aus einer Gruppe umfassend Äpfelsäure, Weinsäure, Bernsteinsäure und/oder Zitronensäure. Es können auch Mischungen verschiedener organischer Säuren eingesetzt werden. Besonders bevorzugt wird Zitronensäure eingesetzt.

Die Temperatur T1 zur physikalischen Deaktivierung, die jedoch auch bei der chemischen Deaktivierung eingesetzt bzw. gehalten werden kann, beträgt über etwa 45°C und liegt üblicherweise in einem Bereich von etwa 48°C bis etwa 55°C. Weiter bevorzugt wird die Messlösung in Schritt b) über einen Zeitraum von mindestens 10 min, weiter bevorzugt über einen Zeitraum von mindestens 10 min bis mindestens 120 min, weiter bevorzugt über einen Zeitraum von mindestens 20 min bis mindestens 90 min, auf der Temperatur T1 gehalten. Die Temperatursteuerung über die Zeit kann ebenfalls automatisiert erfolgen, beispielsweise durch Einsatz von Temperaturfühlern oder sonstigen geeigneten Temperatursensoren. Selbiges gilt auch für die Zudosierung im Rahmen der chemischen Deaktivierung, die ebenfalls vollautomatisiert erfolgen kann.

Erfindungsgemäß erfolgt, bevorzugt in Schritt b.) gleichzeitig oder nachfolgend der chemischen Deaktivierung durch Zugabe eines pH-Wert-Regulierungsmittels, für eine Deaktivierung von in der Messlösung enthaltenen Bakterien eine Zugabe mindestens eines für nicht zu bestimmende thermophile Mikroorganismen spezifischen Breitbandbiozides, für eine Deaktivierung von in der Messlösung vorhandener oxidierender und/oder nichtoxidierender Biozide eine Zugabe mindestens einer Inaktivatorlösung und /oder für eine Deaktivierung von in der Messlösung vorhandener Enzyme eine Zugabe mindestens eines Enzymhemmers. Die vorgenannten zusätzlichen und alternativen Zugaben fallen im Sinne der vorstehenden Erfindung unter den Oberbegriff einer chemischen Deaktivierung der Messlösung.
Die in Schritt b) physikalisch und/oder chemisch zu deaktivierende Begleitflora setzt sich insbesondere aus Bakterien unterschiedlichster Art, aber auch Pilzen u. ä. zusammen und kann als mikrobielle Begleitflora im Sinne der vorliegenden Erfindung verstanden werden. Diese begleitet die zu bestimmenden thermophilen Bakterien, insbesondere die zu bestimmenden lebenden pathogenen thermophilen Bakterien, wie insbesondere Legionella spp., und setzt sich dementsprechend aus sämtlichen sonstigen denkbaren Bestandteilen zusammen wie eben anderen, auch thermophilen Bakterien, Pilzen, Viren, sonstigen Bestandteilen von Mikroben etc. Soweit gezielt ein bestimmtes thermophiles Bakterium bestimmt werden soll, ist erfindungsgemäß eine chemische Deaktivierung, wie bereits vorstehend erwähnt, durch Zugabe, die bevorzugt in Schritt b) erfolgt, mindestens eines für nicht zu bestimmende thermophile Mikroorganismen, zu welchen auch die thermophilen Bakterien zählen, spezifischen Breitbandbiozides erfindungsgemäß vorgesehen. Alternativ oder zusätzlich kann mindestens eine spezifische Inaktivatorlösung für nichtoxidierende und/oder oxidierende Biozide, bevorzugt in Schritt b), eingesetzt werden, da Biozide in wasserführenden Anlagen aufgrund Verunreinigungen vorhanden sein können, die ebenfalls das Messergebnis negativ beeinflussen würden. Zur chemischen Deaktivierung oxidierender Biozide wird vorzugsweise im Sinne der vorliegenden Erfindung mindestens eine sulfithaltige Lösung, insbesondere eine wässrige Lösung aus Natriumthiosulfat und/oder Natriumhydrogenthiosulfat, eingesetzt. Zur Inaktivierung gängiger nichtoxidierender Biozide können alternativ oder zusätzlich mindestens eine weitere oder mehrere Substanzen eingesetzt werden, bei denen es sich beispielsweise um nichtionogene Tenside, Ethylen-/Propylenethoxylate, Lecithin, Saponin, Natriumlaurylsulfonat, L-Histidin, Glycin oder Natriumglykolat handelt. Und schließlich können zur chemischen Deaktivierung von in der Messlösung enthaltenen Enzymen spezifische Enzymhemmer eingesetzt werden, welche extrazelluläre Enzyme blockieren, so dass durch diese eine Beeinflussung des Messergebnisses entfällt. Ein Beispiel eines solchen Enzymhemmers wäre 4-(4-Morpholinyl)-1,2,5-thiadiazol-3-yl N-zyclooctyl-N-Methylcarbamat), welcher irreversibel a/ß-Hydrolase inhibiert.

Sämtliche Zudosierungen der vorgenannten Substanzen können dabei teil- oder vollautomatisiert im erfindungsgemäßen Verfahren erfolgen. Bevorzugt im Sinne der vorliegenden Erfindung wird bei dem erfindungsgemäßen Verfahren in Schritt b) zur chemischen Deaktivierung eine Einstellung des pH-Wertes durch organische Säuren, wie vorstehend beschrieben, und bevorzugt mit Zitronensäure vorgenommen, fakultativ ergänzt durch die Zugabe mindestens einer sulfithaltigen Lösung zur Deaktivierung oxidierender Biozide als auch weiter fakultativ Zugabe mindestens einer spezifischen Inaktivierungslösung für nichtoxidierende Biozide. Ergänzend hierzu kann dann mindestens ein Enzymhemmer und/oder mindestens ein Breitbandbiozid, welches nicht für die zu bestimmenden thermophilen Bakterien spezifisch ist, zudosiert werden. Zur Erhöhung der Messgenauigkeit des erfindungsgemäßen Verfahrens ist dies vorteilhaft. Allerdings ist in Hinblick auf die Zielrichtung des erfindungsgemäßen Verfahrens, einen Schnelltest zur Verfügung zu stellen, der mit dem akkreditiertem Verfahren der Koloniebildung unter Einsatz von Nährboden korreliert, jedoch nicht exakt dessen Ergebnisse liefert, es je nach wasserführender Anlage auch ausreichend, unter Kostengesichtspunkten von der Zudosierung der vorgenannten Substanzen abzusehen.

Erfindungsgemäß erfolgt in Schritt c) des erfindungsgemäßen Verfahrens ein Absenken der Temperatur der Messlösung auf eine Temperatur T2 auf einen Bereich von etwa 38°C bis etwa 48 °C, bevorzugt auf einen Bereich von etwa 40°C bis etwa 46°C, noch weiter bevorzugt auf einen Bereich von etwa 42°C bis etwa 45°C. Das Absenken auf die Temperatur T2 erfolgt dabei bevorzugt passiv durch Abschaltung eines Beheizungsmittels, beispielsweise eines Heizelementes, welches bevorzugt in Schritt b) zur Erzielung der erhöhten Temperatur T1 eingesetzt wird. Durch die deutlich über Raumtemperatur liegende Temperatur T2, die bevorzugt derart gewählt ist, dass diese in einem Bereich liegt, in welchem eine Vielzahl nichtthermophiler Bakterien, bevorzugt die Mehrzahl, weiter bevorzugt nahezu vollständig, degenerieren und/oder inaktiv werden, wird vorteilhafterweise ein Großteil der Begleitflora in wasserführenden Anlagen, insbesondere der mikrobiellen Begleitflora, insbesondere der Begleitflora, welche nicht den zu bestimmenden thermophilen Bakterien entspricht, inaktiviert und kann damit für die Bestimmung und die Auswertung in den Schritten e) und f) des erfindungsgemäßen Verfahrens keinen Einfluss ausüben. Bevorzugt wird zugleich der pH-Wert, der vorzugsweise durch die Zugabe mindestens eines pH-Wert-Regulierungsmittels, insbesondere zumindest einer organischen Säure, bevorzugt Zitronensäure, eingestellt wird, auf einen Wert unter etwa 6,5, bevorzugt in einem Bereich von etwa 5,5 bis etwa 6,5, nicht nur eingestellt, sondern auch in den Schritten b) und/oder c) gehalten, wobei ggf. auch eine Zudosierung des pH-Wert-Regulierungsmittels erfolgt.

Die im Schritt d) erfindungsgemäß vorgesehene Zudosierung mindestens einer Indikatorlösung erfolgt bevorzugt bei einer konstant gehaltenen Temperatur T2 als auch einem konstant gehaltenen pH-Wert in den bevorzugten Bereichen, wie vorstehend wiedergegeben. Dies erfolgt erfindungsgemäß, bevorzugt vollautomatisch, über eine Steuerung, die über eine pH-Wert-Messvorrichtung und ein Temperaturmessmittel mit dem Bioreaktor in Verbindung steht. Das Beheizungsmittel und/oder eine pH-Wert-Regulierungsmitteleinheit, über die das pH-Wert-Regulierungsmittel dem Bioreaktor zudosiert wird, wird hierzu nach- oder eingereglt

Bevorzugt erfolgt die Zudosierung der Indikatorlösung in Schritt d) bei einer Temperatur T2 in einem Bereich von etwa 42°C bis etwa 45°C und bei einem pH-Wert in einem Bereich von etwa 5,5 bis etwa 6,5, wobei der pH-Wert als auch die Temperatur bevorzugt fortlaufend über entsprechende Sensoren überwacht und über eine automatisierte Steuerung dafür gesorgt wird, dass während der Zudosierung im Schritt d), aber weiter vorteilhaft auch während der spektrometrischen Bestimmung im Schritt e), der pH-Wert und die Temperatur T2 konstant gehalten bzw. auf die gewünschten Werte nachgeregelt werden. Bereits hierdurch basiert ein in Schritt e) zu bestimmender Messwert W1 und W2 nahezu vollständig auf thermophilen Bakterien, insbesondere pathogene thermophile Bakterien, wie Legionella spp.

Die in Schritt d) zudosierte Indikatorlösung ist vorzugsweise eine Farbindikatorlösung. Bevorzugt wird eine Farbindikatorlösung eingesetzt, die mindestens ein Indikatormittel umfasst. Weiter bevorzugt ist das mindestens eine Indikatormittel ein solches, welches mikrobiologisch umgesetzt wird durch die Aktivität der zu bestimmenden lebenden thermophilen Bakterien. Die beim erfindungsgemäßen Verfahren einsetzbaren Farbindikatorlösungen sind bevorzugt ausgewählt aus solchen, welche durch mikrobiologischen Umsatz mindestens eines Indikatormittels eine Fluoreszenz erzeugen. Geeignete Indikatormittel, die von den Indikatorlösungen umfasst sind, sind beispielsweise Derivate des Fluorescein. Besonders bevorzugt sind mindestens ein Fluoresceinester und/oder mindestens ein Derivat eines Fluoresceinesters, wie beispielsweise Fluoresceindibutyrat (FDB), Fluoresceindiacetat (FDA) oder dessen Derivat Carboxyfluoresceindiacetat (cFDA). Aber auch weitere Derviate wie 2',7'-Dichlorolfluoresceindiaceat, 5(6)-Carboxyfluoresceindiacetat, 5(6)-Carboxyfluoresceindiacetat-N-succinimidylester, 5-Carboxyfluorescein-N-succinimidylester, 6-Carboxyfluoresceindiacetat-N-succinimidylester, 6-(Fluorescein-5(6)-carboxoamido]-hexanonsäure-N-succinimideester und/oder 6-Carboxy-4',5'-dichloro-2',7'-dimethoxyfluorescein-N-hydroxysuccinimidester können eingesetzt werden. Bevorzugt ist das mindestens eine Indikatormittel in mindestens einen organischen Lösemittel gelöst. Das mindestens eine Lösemittel und das mindestens eine Indikatormittel bilden zusammen die Indikatorlösung. Die Indikatorlösung selbst kann dabei ggf. noch weitere zusätzliche Substanzen, insbesondere Stabilisationsmittel u. ä. enthalten. Besonders bevorzugt wird ein organisches Lösemittel für das mindestens eine Indikatormittel eingesetzt, besonders bevorzugt Aceton. Aber auch weitere Lösemittel wie beispielsweise Dimetylformamid (DMF) oder Dimetylsulfoxid (DMSO) können eingesetzt werden.

Besonders bevorzugt wird im erfindungsgemäßen Verfahren eine Kühlung der zuzudosierenden Indikatorlösung vorgesehen. Eine solche Kühlung erfolgt vorzugsweise bei einer Temperatur in einem Bereich von etwa -15°C bis etwa 6°C, weiter bevorzugt in einem Bereich von - 12°C bis etwa +5°C, noch weiter bevorzugt in einem Bereich von etwa -10°C bis etwa +4°C. Hierdurch wird einer Reaktion bzw. Zersetzung des eingesetzten Indikatormittels in der Indikatorlösung entgegengewirkt. Indikatormittel neigen oftmals zu einer Eigenzersetzung, so auch Fluorescein und dessen Derivate. Hierdurch würde es zu einem möglichen Mehrbefund bei der Bestimmung und der Auswertung gemäß den Schritten e) und f) nach dem erfindungsgemäßen Verfahren kommen. Eine Kühlvorrichtung zur Kühlung der Indikatorlösung kann dabei in einer Vorrichtung, mit der das erfindungsgemäße Verfahren durchgeführt wird, integriert sein, oder aber auch separat hiervon ausgebildet sein. Es kann dann vorgesehen sein, dass eine Pumpe, über welche die Indikatorlösung dem Bioreaktor zugeführt wird, ebenfalls mit einer Kühlung versehen ist, und/oder dass der gesamte Leitungsweg von einem Vorratsgefäß für die Indikatorlösung bis zum Bioreaktor, der eine Indikatorzuführeinheit bildet, isoliert ist, um eine vorzeitige Erwärmung der Indikatorlösung zu vermeiden. Sowohl die Zudosierung der mindestens einen Indikatorlösung in Schritt d) als auch die vorstehend angesprochene Kühlung der mindestens einen Indikatorlösung kann dabei zeit- und volumengesteuert teil- bzw. vollautomatisiert über eine getrennte Steuerung erfolgen. Bevorzugt erfolgt die Zudosierung stoßweise, so dass nicht die gesamte mindestens eine Indikatorlösung in Schritt d) zudosiert wird, sondern dass hierfür mehrere Unterschritte vorgesehen sind. Hierdurch kann eine gleichmäßigere Verteilung der Indikatorlösung erfolgen.

Im Übrigen kann über den gesamten Zeitraum, beginnend mit der Beschickung des Bioreaktors mit der wässrigen Messlösung in Schritt a) bis zur Bestimmung in Schritt e) die Messlösung gerührt werden. Dies kann beispielsweise durch einen üblichen Magnetrührfisch und ein entsprechendes Rührgerät erfolgen. Das Rühren kann dabei auch automatisiert vorgenommen werden.

Erfindungsgemäß erfolgt in Schritt e) des erfindungsgemäßen Verfahrens eine spektrometrische Bestimmung mindestens zweier zu unterschiedlichen Zeitpunkten ermittelter Messwerte W1 und W2 der Messlösung, wobei diese durch mikrobiologischen Umsatz der Indikatorlösung umfassend mindestens ein Indikatormittel bestimmbar sind. Bevorzugt erfolgt bei Einsatz mindestens einer Farbindikatorlösung eine Änderung eines Farbspektrums in Schritt e) bei mindestens einer Wellenlänge, bevorzugt bei Einsatz von Fluoresceinestern und deren Derivaten bei einer Wellenlänge in einem Bereich von 489 nm bis 506 nm, bevorzugt von 496 nm. Besonders bevorzugt werden mehrere Messwerte Wₙ ermittelt. Besonders bevorzugt werden etwa alle 5 min, weiter bevorzugt etwa alle 10 min, weiter bevorzugt etwa alle 15 min Messwerte Wₙ spektrometrisch bestimmt. Bevorzugt erfolgt die spektrometrische Bestimmung bei Einsatz einer Farbindikatorlösung durch ein Fluorometer. Weiter bevorzugt erfolgt ein Beginn der Bestimmung in Schritt e) bei einem vorgegebenen Zeitpunkt nach vollständiger Zudosierung der mindestens einen Indikatorlösung, beispielsweise mindestens etwa 5 sec, weiter bevorzugt mindestens etwa 10 sec nach vollständiger Zudosierung der mindestens einen Indikatorlösung. Die spektrometrische Bestimmung in Schritt e) erfolgt bevorzugt über einen Zeitraum von mindestens etwa 10 min, weiter bevorzugt über einen Zeitraum von mindestens etwa 30 min und erfolgt weiter bevorzugt in einem Bereich zwischen etwa 30 min und etwa 240 min, und noch weiter bevorzugt in einem Bereich zwischen etwa 45 min und etwa 200 min.

Aus den im Schritt e) bestimmten mindestens zwei Messwerten W1 und W2, vorteilhafterweise mehreren Messwerten Wₙ, wie vorstehend beschrieben, wird in Schritt f) zur Auswertung bevorzugt eine Steigung der Messwerte des Spektrometers bestimmt. Diese Steigung korreliert mit den koloniebildenden Einheiten bei dem akkreditiertem Nährbodenverfahren, wie eingangs beschrieben, und damit den lebenden und aktiven thermophilen Bakterien. Die solchermaßen ermittelte Konzentration in Schritt f) der zu bestimmenden lebenden thermophilen Bakterien ist das Ergebnis des erfindungsgemäßen Verfahrens. Da zudem bevorzugt eine Messdauer vorgesehen ist, innerhalb welcher keine weitere Zellteilung der zu bestimmenden lebenden thermophilen Bakterien erfolgt, kann hierdurch sichergestellt werden, dass das Messergebnis nicht weiter verfälscht wird und in der Tat nur die lebenden thermophilen Bakterien in der Messlösung bestimmt werden. Besonders bevorzugt erfolgt daher die spektrometrische Bestimmung in Schritt e) des erfindungsgemäßen Verfahrens über einen Zeitraum von nicht über etwa 240 min.

Zudem ist eine spektrometrische Bestimmung in Schritt e) des erfindungsgemäßen Verfahrens auch möglich bei feststoffhaltigen, verschmutzten, beispielsweise öl- und/oder graphitbelasteten Flüssigkeiten aus wasserführenden Anlagen, die dementsprechend auch in der Messlösung vorliegen. Denn eine Ausgangstrübung oder -färbung der Messlösung beeinflusst die Bestimmung in Schritt e) nur unwesentlich. Vorteilhafterweise wird vor Zugabe der mindestens einen Indikatorlösung in Schritt d) das Grundsignal der Messlösung als Nullwert ermittelt, so dass ein entsprechendes Grundsignal auch aus den Messwerten W1 und W2 bzw. den weiteren Messwerten Wₙ herausgerechnet werden kann.

Die Auswertung der bestimmten mindestens zwei Messwerte W1 und W2 bzw. der weiteren Messwerte Wₙ gemäß Schritt f) erfolgt, wie vorstehend bereits erwähnt, aus der Ermittlung der Steigung des Messsignales über einen bestimmten Zeitraum. Erfindungsgemäß wird in Schritt e) hierzu ein Messwert W1 in einem Bereich von etwa 30 min bis etwa 90 min bestimmt jeweils beginnend ab dem Ende der Zudosierung der Indikatorlösung in Schritt d). Weiter bevorzugt wird in Schritt e) ein Messwert W2 in einem Bereich von etwa 120 min bis etwa 210 min bestimmt, jeweils beginnend ab dem Ende der Zudosierung der Indikatorlösung in Schritt d). Aus der hieraus bzw. in noch weiteren Messwerten Wₙ ermittelten Steigung kann dann über eine Kalibrierformel eine Konzentration lebender thermophiler Bakterien ermittelt werden. Hierfür sind zumindest zwei Messwerte W1 und W2 notwendigerweise zu bestimmen, eine Erhöhung der Mehrzahl der Messwerte Wₙ führt dabei zu einer weitergehenden Genauigkeit der ermittelbaren Konzentration lebender thermophiler Bakterien, insbesondere lebender pathogener thermophiler Bakterien, wie insbesondere Legionella spp. Aufgrund der kurzen Dauer des erfindungsgemäßen Verfahrens wird vorteilhafterweise die Aktivität nur der vorhandenen lebenden thermophilen Bakterien bestimmt und nicht solche lebenden thermophilen Bakterien mithinzugerechnet, die durch Zellteilung entstehen. Hierdurch ist es dem Benutzer des erfindungsgemäßen Verfahrens möglich, bei seiner wasserführenden Anlage, beispielsweise bei Kühlwasserkreisläufen, schnell auf eventuelle Probleme zu reagieren.

Nachfolgend dem Schritt e) oder f) wird erfindungsgemäß eine Desinfektion der für die Durchführung des erfindungsgemäßen Verfahrens eingesetzten Vorrichtung mit Messlösung mit Indikatorlösung bei einer Temperatur von über etwa 63°C, weiter bevorzugt über etwa 70°C, vorgenommen. Bevorzugt erfolgt die Desinfektion bei einer Temperatur in einem Bereich von etwa 63°C bis etwa 80°C, weiter bevorzugt bei einer Temperatur in einem Bereich von etwa 68°C bis etwa 76°C. Hierdurch kann dann die Vorrichtung, mittels der das erfindungsgemäße Verfahren durchgeführt wird, wieder in einen Zustand versetzt werden, in dem eine neue bzw. weitere Messung der Konzentration lebender thermophiler Bakterien in wasserführenden Anlagen ermöglicht ist. Diese neue bzw. weitere Messung kann dabei auch zeitgesteuert automatisiert erfolgen. Für die Desinfektion kann dabei vorgesehen sein, zwischen der ersten Regeleinheit und der zweiten Regeleinheit die in dem Bioreaktor und der Zuführleitung und der Abführleitung vorhandene Messlösung im Kreislauf zu führen und damit den Bioreaktor und die Zuführleitung und Abführleitung vollständig zu desinfizieren. Hierzu kann mindestens eine Pumpe vorgesehen sein, die in einer Verbindungsleitung zwischen Abführleitung und Zuführleitung des Bioreaktors nach der ersten Regeleinheit und vor der zweiten Regeleinheit angeordnet ist. Zudem kann auch zum Zwecke der Desinfektion ein weiterer Kreislauf vorgesehen sein, in dem nachfolgend der zweiten Regeleinheit und vor der ersten Regeleinheit in der Abführleitung bzw. Zuführleitung eine weitere Verbindungsleitung mit einer dort angeordneten Pumpe vorgesehen ist, so dass hier fakultativ auch ein Bereich unmittelbar vor oder nach der ersten Regeleinheit und der zweiten Regeleinheit desinfizierbar ist. Hierzu können dann ergänzend weitere Regeleinheiten in der Vorrichtung, mittels welcher das erfindungsgemäße Verfahren durchgeführt wird, vorgesehen sein, die in der Regel als Absperrorgane, insbesondere als Ventile, ausgebildet sind. In der Vorrichtung wird die Vermehrung insbesondere von thermophilen und möglicherweise pathogenen Bakterien, wie beispielsweise Legionella pneumophila, vermieden. Die möglicherweise in der Vorrichtung enthaltenen pathogenen Bakterien werden im Desinfektionsschritt abgetötet und nach dem Desinfektionsschritt aus der Vorrichtung gefahrlos abgelassen.

Besonders bevorzugt erfolgt die Bestimmung der Konzentration lebender thermophiler Bakterien, bevorzugt lebender pathogener thermophiler Bakterien, wie insbesondere Legionella spp., vollautomatisiert in zeitlich vorgegebenen Intervallen. Wie bereits vorstehend erwähnt, kann sowohl die Steuerung der ersten Regeleinheit und der zweiten Regeleinheit in der Zuführleitung bzw. Abführleitung zum Bioreaktor zeitlich gesteuert werden, als auch die Befüllung des Bioreaktors unter Verwendung eines Füllstandsensors. Ebenso kann die Einstellung eines pH-Wertes über eine pH-Sonde und damit eine chemische Deaktivierung ebenso wie eine physikalische Deaktivierung über ein Beheizungsmittel, beispielsweise ein Heizelement, und eine Heizsonde bzw. einen Temperatursensor vollautomatisiert ermittelt und ggf. nachgeregelt werden, ebenso wie die Zudosierung des für die pH-Wert-Einstellung notwendigen mindestens einen pH-Wert-Regulierungsmittels, insbesondere mindestens einer organischen Säure, als auch der sonstigen Substanzen wie Breitbandbioziden, Inaktivatorlösungen, Enzymhemmern etc.,. Bevorzugt ist eine Steuerung, bei welcher die entsprechenden Informationen zusammenlaufen bzw. Regelungen vorgenommen werden, mit einem Computer bzw. einem Datenfernübertragungsmittel wie einem Modem o. ä. verbunden. Hierdurch wäre auch eine Fernwartung des erfindungsgemäßen Verfahrens vollautomatisiert ermöglicht.

Die vorliegende Erfindung betrifft weiterhin eine Vorrichtung zur Durchführung eines erfindungsgemäßen Verfahrens mit einem Bioreaktor mit einer wasserführenden Zuführleitung mit mindestens einer ersten Regeleinheit für eine wässrige Messlösung und eine Abführleitung mit mindestens einer zweiten Regeleinheit, weiterhin umfassend eine pH-Wert-Messvorrichtung, die mit der Messlösung im Bioreaktor während einer Messung in Kontakt steht, einer pH-Wert-Regulierungsmitteleinheit zur Zuführung des Regulierungsmittels in den Bioreaktor, ein Beheizungsmittel für die Messlösung, welches bevorzugt mit dieser in Kontakt steht, mindestens eine Indikatorzuführeinheit in den Bioreaktor, mindestens ein Spektrometer und eine rechnergestützte Auswerteeinheit zur Ermittlung der Konzentration lebender thermophiler Bakterien. Weiter bevorzugt umfasst die erfindungsgemäße Vorrichtung einen Temperatursensor, der die Temperatur im Bioreaktor während der Messung durch spektrometrisches Bestimmen von mindestens zwei Messwerten W1 und W2, aber auch im Schritt b) betreffend die physikalische Deaktivierung als auch betreffend die Absenkung der Temperatur in Schritt c) bestimmt und bevorzugt diese Information einer Steuereinheit übergibt. Diese Steuereinheit, die von der erfindungsgemäßen Vorrichtung umfasst ist, kann dann je nach Bedarf das Beheizungsmittel für die Messlösung regeln.

Weiter bevorzugt umfasst die erfindungsgemäße Vorrichtung eine Rühreinheit zum Rühren der Messlösung mit und ohne Indikatorlösung. Bevorzugt ist die Rühreinheit als Magnetrührer mit einem Rührfisch ausgebildet. Aber auch jede andere Art der Ausbildung der Rühreinheit ist möglich. Ein Rühren der Messlösung erfolgt durch die Rühreinheit bevorzugt zumindest bei Durchführung einer chemischen Deaktivierung, und erfolgt über die Zudosierung der mindestens einen Indikatorlösung hinaus weiter bevorzugt auch während der spektrometrischen Bestimmung der mindestens zwei Messwerte W1 und W2.

Weiter bevorzugt umfasst die mindestens eine Indikatorzuführeinheit der erfindungsgemäßen Vorrichtung mindestens eine Dosiervorrichtung für die Zuführung der mindestens einen Indikatorlösung in den Bioreaktor. Diese Dosiervorrichtung ist vorteilhafterweise als Pumpe ausgebildet.

Das mindestens eine von der erfindungsgemäßen Vorrichtung umfasste Spektrometer ist bevorzugt ein Fluorometer. Dieses ist bevorzugt derart ausgebildet, dass es eine Fluoreszenz des mindestens einen Indikatormittels in der mindestens einen Indikatorlösung nachweisen kann. Die mindestens eine Indikatorlösung umfasst bevorzugtmindestens einen Fluoresceinester und/oder mindestens ein Derivat eines Fluoresceinesters. Die Indikatorzuführeinheit umfasst weiter vorteilhafterweise eine Kühlungseinheit zur Einstellung einer Temperatur der Indikatorlösung in einem Bereich von etwa -15°C bis etwa 6°C, weiter bevorzugt in einem Bereich von etwa -10°C bis etwa 5°C. Erfindungsgemäß erfolgt eine Auswertung in Schritt f) durch eine rechnergestützte Auswerteeinheit, entweder vor Ort oder aber über eine Fernauswertung bei einem Drittanbieter, wobei die Konzentration lebender thermophiler Bakterien ermittelt wird. Weiter vorteilhafterweise umfasst die erfindungsgemäße Vorrichtung weiterhin mindestens eine Biozidzuführeinheit, insbesondere zur Zuführung eines Breitbandbiozides, mindestens eine Enzymhemmerzuführeinheit und/oder mindestens eine Inaktivatorzuführeinheit für oxidierende Biozide und/oder für nichtoxidierende Biozide. Die mindestens eine Biozidzuführeinheit für Breitbandbiozide ist derart ausgestaltet, dass ein Breitbandbiozid zugeführt wird, welches nicht für zu bestimmende lebende thermophile Mikroorganismen spezifisch ist.

Wenn im Rahmen der Erfindung der Begriff "etwa" oder "im Wesentlichen" in Bezug auf Werte oder Wertebereiche verwendet wird oder ergeben sich bei der Verwendung dieser Begriffe aus dem Zusammenhang bestimmte Werte, ist hierunter dasjenige zu verstehen, was der Fachmann in dem gegebenen Zusammenhang als fachmännisch üblich ansehen wird. Insbesondere sind Abweichungen der angegebenen Werte von +/- 10 %, bevorzugt von +/- 5 %, weiter bevorzugt von +/- 2 %, besonders bevorzugt von +/- 1 %, von den Begriffen "etwa" und "im Wesentlichen" umfasst.

Mit dem erfindungsgemäßen Verfahren und der erfindungsgemäßen Vorrichtung kann eine Bestimmung der Konzentration lebender thermophiler Bakterien, insbesondere pathogener lebender thermophiler Bakterien, wie insbesondere Legionella spp., in einem Zeitraum von weniger als etwa 240 min durchgeführt werden. Hierdurch erhält der Betreiber einer wasserführenden Anlage schnell eine Information über mögliche Maßnahmen, die er ergreifen muss. Zudem erhält er auch in Hinblick auf die ermittelbare Konzentration Aussagen, in welchem Umfang und in welcher Menge er Mittel einsetzen muss, um lebende thermophile Bakterien in der wasserführenden Anlage zu beseitigen. Durch diese Information kann der Betreiber einer wasserführenden Anlage nicht unerheblich Kosten durch Einsparungen bei den entsprechenden Mitteln aufgrund einer genaueren möglichen Mengenbestimmbarkeit erzielen. Da zudem die Durchführung des erfindungsgemäßen Verfahrens mit der erfindungsgemäßen Vorrichtung in einem Zeitraum erfolgt, in welchem noch keine Zellteilung lebender thermophiler Bakterien erfolgt, kann insofern hierdurch auch eine recht genaue Bestimmung der Konzentration selbiger erfolgen.

Diese und weitere Vorteile der vorliegenden Erfindung werden anhand der folgenden Figur näher erläutert. Es zeigt:

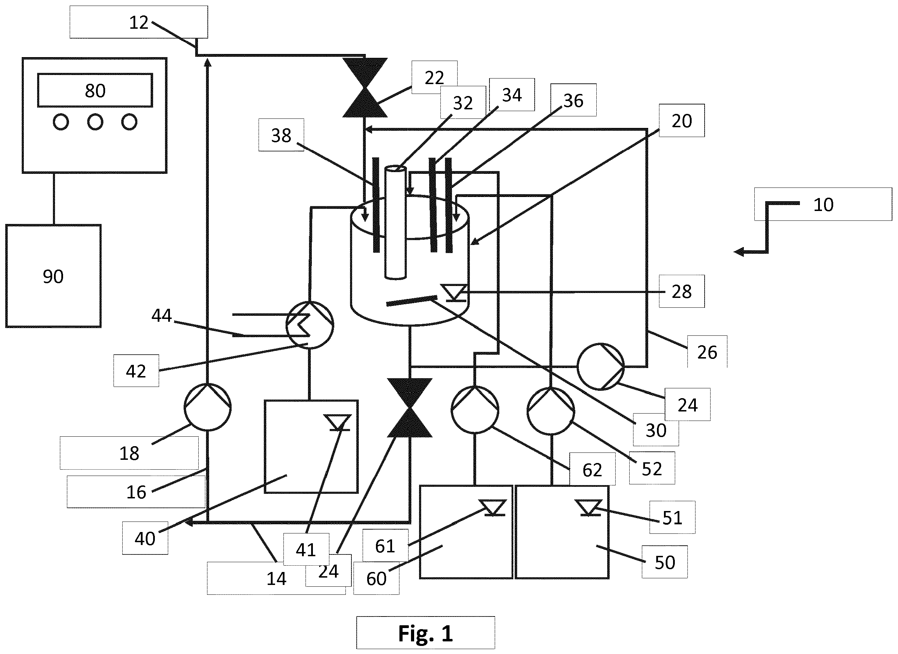

- Fig. 1:: eine schematische Skizze einer erfindungsgemäßen Vorrichtung zur Durchführung eines erfindungsgemäßen Verfahrens.

Fig. 1 zeigt eine erfindungsgemäße Vorrichtung 10 zur beschleunigten Bestimmung der Konzentration lebender thermophiler Bakterien, insbesondere pathogener lebender thermophiler Bakterien, wie Legionella spp., mit einem Bioreaktor 20. Aus einer hier nicht näher gezeigten wasserführenden Anlage wird über eine Zuführleitung 12 und eine erste Regeleinheit 22, welche als Ventil ausgebildet ist, eine Messlösung in den Bioreaktor 20 geleitet. Die Messlösung kann aus dem Bioreaktor 20 über eine Abführleitung 14 über eine zweite Regeleinheit 24, welche ebenfalls als Ventil ausgebildet ist, abgeführt werden. Soll der Bioreaktor mit wässriger Messlösung aus der wasserführenden Anlage beschickt werden, öffnet sich das Ventil 22, so dass Messlösung über die Zuführleitung 12 in den Bioreaktor 20 strömt. Das zweite Ventil 24 ist dabei geschlossen oder wird nach einer gewissen Zeit geschlossen. Wird im Bioreaktor 20 ein vorgesehener Füllstand, ermittelbar über einen Füllstandssenor 28, erreicht, wird dann das Ventil 22 geschlossen. Öffnung und Schließung der Ventile 22 und 24 kann dabei vollautomatisch gesteuert über eine Steuereinheit 80, die schematisch in Fig. 1 gezeigt ist, erfolgen, welche auch mit dem Füllstandsensor 28 verbunden ist und nach Erreichen der Füllhöhe im Bioreaktor 20 das Ventil 22 sperrt.

Im Bioreaktor 20 angeordnet und in Kontakt mit der in diesem vorhandenen Messlösung ist ein Temperatursensor 34, ein Heizelement 36 als auch eine pH-Wert-Messvorrichtung 38 sowie ein Fluorometer 32. Mit dem Bioreaktor 20 verbunden ist über eine nicht näher bezeichnete Zuführleitung eine Indikatorzuführeinheit umfassend einen Indikatorlösungsbehälter 40 mit der Indikatorlösung und einen in diesem angeordneten Füllstandsensor 41, wobei in der Zuführleitung zum Bioreaktor 20 eine Pumpe 42 mit einer Kühlung 44 vorgesehen ist. Der Indikatorlösungsbehälter 40 kann dabei vorteilhafterweise über ein Peltierelement ebenso gekühlt werden wie die Zuführleitung zum Bioreaktor 20, um einer Eigenzersetzung mindestens eines in dem Indikatorlösungsbehälter 40 vorhandenen Indikatormittels vorzubeugen.

Weiterhin ist der Bioreaktor 20 verbunden mit einer pH-Wert-Regulierungsmitteleinheit, welche einen pH-Wert-Regulierungsmittelbehälter 50 mit einem Füllstandsensor 51 und eine in einer nicht näher bezeichneten Zuführleitung zum Bioreaktor 20 angeordneten Pumpe 52 umfasst. Des Weiteren ist eine Inaktivatorzuführeinheit für beispielsweise eine Lösung von Natriumthiosulfat für oxidierende Biozide und/oder für eine Formulierung mindestens eines organischen Inaktivators für nichtoxidierende Biozide vorgesehen, die einen Inaktivatorzuführbehälter 60 mit einem entsprechenden Füllstandsensor 61 und eine in einer nicht näher bezeichneten Zuführleitung angeordnete Pumpe 62 umfasst. Die Inaktivatorzuführeinheit, die pH-Wert-Regulierungsmitteleinheit als auch die Indikatorzuführeinheit, umfassend den Indikatorlösungsbehälter 40 mit Füllstandsensor 41, Pumpe 42, Kühlung 44 und die entsprechend Zuführleitung zum Bioreaktor 20, sind dabei über die Steuerung 80 ansteuerbar. Diese steuert die Zugabe der Indikatorlösung, des pH-Wert-Regulierungsmittels als auch der Inaktivator-haltigen Lösung über die Pumpen 42, 52 und 62, wobei insbesondere in Abhängigkeit von der pH-Wert-Messvorrichtung 38 ein pH-Wert der Messlösung im Messbehälter 20 während der spektrometrischen Bestimmung durch das Fluorometer 32 konstant gehalten wird. Die Steuereinheit 80 ist im Übrigen mit einer in Fig. 1 schematisch bezeichneten Datenfernübertragungseinrichtung 90 mit einem Fernwartungsrechner verbindbar, über welchen eine Steuerung der Vorrichtung 10 zur Durchführung des erfindungsgemäßen Verfahrens erfolgt.

Des Weiteren umfasst die Vorrichtung 10 eine nur schematisch wiedergegebene Magnet-Rühreinheit 30 mit einem Rührfisch, der die Messlösung im Bioreaktor 20 zumindest mit Beginn der physikalischen und/oder chemischen Deaktivierung und über die Zudosierung der mindestens einen Indikatorlösung als auch ggf. der spektrometrischen Bestimmung selbst rührt.

Zum Zwecke der Desinfektion nach spektrometrischer Bestimmung über das Fluorometer 32 als auch Auswertung der damit erhaltenen mindestens zwei Messwerten W1 und W2, bevorzugt mehrerer Messwerte Wₙ, ist eine Verbindungsleitung 26 mit einer Pumpe 24 vorgesehen, welche die Messlösung nach Auswertung im Kreislauf zwischen den Ventilen 22 und 24 führt. Des Weiteren ist in Fig. 1 ein weiterer Desinfizierungskreislauf vorgesehen mit einer Verbindungsleitung 16 zwischen der Abführleitung 14 und der Zuführleitung 12 und einer in der Leitung 16 vorgesehen Pumpe 18. Hier nicht dargestellt sind weitere, nachfolgend den Angreifpunkten der Leitung 16 an der Abführleitung 14 und der Zuführleitung 12 angeordnete Absperrorgane in Form von Ventilen.

Mittels des Beheizungsmittels 36, welches in Form beispielsweise eines Heizstabes ausgebildet sein kann, wird zunächst eine physikalische Deaktivierung bei beispielsweise einer Temperatur von 50°C durchgeführt, wobei über den Temperatursensor 34 und die Steuerung 80 dies überwacht wird. Dabei kann die Temperatur der Messlösung beispielsweise über 45 min automatisiert bei 50°C im Bioreaktor 20 gehalten werden. Anschließend kann die Temperatur passiv durch Ausschalten des Beheizungsmittels 36 auf eine Temperatur T2 von beispielsweise 43°C eingestellt werden, wobei wiederum über den Temperatursensor 34 dies in Verbindung mit der Steuerung 80 überwacht und eine Regelung auf diese Temperatur T2 vorgenommen wird. Eine Temperatur T 2 von beispielsweise 43°C kann dann über den Zeitraum der Zudosierung der mindestens einen Indikatorlösung über die Indikatorzuführeinheit aus dem Indikatorlösungsbehälter 40 bis zum Abschluss der spektrometrischen Bestimmung mittels des Fluorometers 32 gehalten werden. Ebenso kann dann nach spektrometrischer Bestimmung oder aber nach Auswertung mittels des Beheizungsmittels 36 die Desinfektion bei beispielsweise 70°C vorgenommen werden, damit die erfindungsgemäße Vorrichtung 10 für eine weitere, neue Messung zur Verfügung steht.

Im Bioreaktor 20 wird während der Zugabe der Indikatorlösung als auch während der spektrometrischen Bestimmung mittels des Fluorometers 32 nicht nur die Temperatur T2, wie vorstehend beschrieben, konstant gehalten, sondern auch der pH-Wert durch die pH-Wert-Messvorrichtung 38, die ebenfalls mit der Steuerung 80 in Verbindung steht. Hierzu kann dann aus der pH-Wert-Regulierungsmitteleinheit z. B. Zitronensäure in wässriger Lösung aus dem Behälter 50 über die Pumpe 52 dem Bioreaktor, auch nachregelnd, zudosiert werden. Nach Zugabe der Indikatorlösung wird über das Fluorometer 32 die Konzentration lebender thermophiler Bakterien bestimmt, indem zumindest zwei Messwerte W1 und W2, bevorzugt mehrere Messwerte Wₙ ermittelt und aus einer Steigung der Messwertsignale letztendlich über eine Kalibrierungsformel eine Konzentration lebender thermophiler Bakterien ermittelt wird. Der Betreiber einer wasserführenden Anlage, an oder in welche die erfindungsgemäße Vorrichtung 10 gemäß Fig. 1 beispielsweise über Flanschverbindungen angebunden ist, kann hierdurch schnell die geeigneten Maßnahmen bei auftretenden Problemen ergreifen.

Die erfindungsgemäße Vorrichtung und das erfindungsgemäße Verfahren sind nicht beschränkt ausschließlich auf die Bestimmung von Legionella spp. bzw. pathogener lebender thermophiler Bakterien. Auch sonstige lebende thermophile Bakterien können mit dem erfindungsgemäßen Verfahren und der erfindungsgemäßen Vorrichtung bestimmt werden.

## Patentansprüche

1. Verfahren zur beschleunigten Bestimmung der Konzentration lebender thermophiler Bakterien in wasserführenden Anlagen, insbesondere in Kühlwasserkreisläufen, bei welchem ein Bioreaktor (20) mit einer wasserführenden Zuführleitung (12) mit mindestens einer ersten Regeleinheit (22) für eine wässrige Messlösung und einer Abführleitung (14) mit mindestens einer zweiten Regeleinheit (24) verbunden ist, umfassend die Schritte
a) Beschicken des Bioreaktors (20) mit der wässrigen Messlösung;
b) physikalische und chemische Deaktivierung von in der Messlösung enthaltener Begleitflora, Bioziden und/oder Enzymen bei einer erhöhten Temperatur T1 über einen Zeitraum von mindestens etwa 1 min, wobei die physikalische und/oder chemische Deaktivierung derart erfolgt, dass die zu bestimmenden thermophilen Bakterien nicht deaktiviert werden;
c) Absenken der Temperatur der Messlösung auf eine Temperatur T2 auf einen Bereich von etwa 38°C bis etwa 45 °C;
d) Zudosieren mindestens einer Indikatorlösung;
e) nach Durchführen der Schritte a) bis d) spektrometrisches Bestimmen mindestens zweier zu unterschiedlichen Zeitpunkten ermittelter Messwerte W1 und W2 der Messlösung mit einer Indikatorlösung, umfassend mindestens ein Indikatormittel, durch mikrobiologischen Umsatz des mindestens einen Indikatormittels; und
f) Auswerten der bestimmten mindestens zwei Messwerte W1 und W2 und Ermitteln einer Konzentration der lebenden thermophilen Bakterien in der Messlösung.

2. Verfahren gemäß Anspruch 1, **dadurch gekennzeichnet, dass** die physikalische Deaktivierung in Schritt b) bei einer Temperatur T1 über 45°C durchgeführt wird.

3. Verfahren gemäß einem oder mehreren der vorhergehenden Ansprüche, **dadurch gekennzeichnet, dass** die chemische Deaktivierung zumindest über eine Einstellung des pH-Wertes der Messlösung auf einen Wert unter etwa 6,5 vorgenommen wird.

4. Verfahren gemäß einem oder mehreren der vorhergehenden Ansprüche, **dadurch gekennzeichnet, dass** die Deaktivierung von in der Messlösung enthaltenen Bakterien durch eine Zugabe mindestens eines für nicht zu bestimmende thermophile Mikroorganismen spezifischen Breitbandbiozides, die Deaktivierung von in der Messlösung vorhandenen oxidierenden und/oder nichtoxidierenden Bioziden durch Zugabe mindestens einer Inaktivatorlösung und/oder die Deaktivierung von in der Messlösung vorhandenen Enzymen durch Zugabe mindestens eines Enzymhemmers erfolgt.

5. Verfahren gemäß einem oder mehreren der vorhergehenden Ansprüche, **dadurch gekennzeichnet, dass** die Indikatorlösung in Schritt d) eine Farbindikatorlösung ist, wobei eine Änderung eines Farbspektrums der Farbindikatorlösung in Schritt e) bei mindestens einer Wellenlänge bestimmt wird.

6. Verfahren gemäß einem oder mehreren der vorhergehenden Ansprüche, **dadurch gekennzeichnet, dass** in Schritt e) ein Messwert W1 in einem Bereich von etwa 30 min bis etwa 90 min nach Beendigung der Zudosierung gemäß Schritt d) bestimmt wird.

7. Verfahren gemäß einem oder mehreren der vorhergehenden Ansprüche, **dadurch gekennzeichnet, dass** in Schritt e) ein Messwert W2 in einem Bereich von etwa 120 min bis etwa 210 min nach Beendigung der Zudosierung gemäß Schritt d) bestimmt wird.

8. Verfahren gemäß einem oder mehreren der vorhergehenden Ansprüche, **dadurch gekennzeichnet, dass** nach Schritt e) oder f) eine Desinfektion der Messlösung mit Indikatorlösung und einer für die Durchführung des Verfahrens eingesetzten Vorrichtung bei einer Temperatur über etwa 63°C vorgenommen wird.

9. Verfahren gemäß einem oder mehreren der vorhergehenden Ansprüche, **dadurch gekennzeichnet, dass** die Bestimmung der Konzentration lebender thermophiler Bakterien automatisiert in zeitlich vorgegebenen Intervallen durchgeführt wird.

10. Vorrichtung (10) zur Durchführung eines Verfahrens gemäß einem oder mehreren der vorhergehenden Ansprüche mit einem Bioreaktor (20) mit einer wasserführenden Zuführleitung (12) mit mindestens einer ersten Regeleinheit (22) für eine wässrige Messlösung und einer Abführleitung (14) mit mindestens einer zweiten Regeleinheit (24), weiterhin umfassend eine pH-Wert-Messvorrichtung (34), die mit der Messlösung im Bioreaktor (20) während einer Messung in Kontakt steht, einer pH-Wert-Regulierungsmitteleinheit (50) zur Zuführung eines Regulierungsmittels in den Bioreaktor (20), ein Beheizungsmittel (38) für die Messlösung, mindestens eine Indikatorzuführeinheit (40) für die Zuführung einer Indikatorlösung in den Bioreaktor (20), mindestens ein Spektrometer (32) und eine rechnergestützte Auswerteeinheit (90) zur Ermittlung der Konzentration lebender thermophiler Bakterien.

11. Vorrichtung gemäß Anspruch 10, **dadurch gekennzeichnet, dass** diese weiterhin eine Rühreinheit (30) zur Rührung der Messlösung mit und ohne Indikatorlösung umfasst.

12. Vorrichtung gemäß einem oder mehreren der Ansprüche 10 bis 11, **dadurch gekennzeichnet, dass** die mindestens eine Indikatorzurführeinheit (40) mindestens eine Dosiervorrichtung (42) für die Zuführung der mindestens einen Indikatorlösung in den Bioreaktor (20) umfasst.

13. Vorrichtung gemäß einem oder mehreren der Ansprüche 10 bis 12, **dadurch gekennzeichnet, dass** das Spektrometer (32) ein Fluorometer ist.

14. Vorrichtung gemäß einem oder mehreren der Ansprüche 10 bis 13, **dadurch gekennzeichnet, dass** die mindestens eine Indikatorlösung mindestens einen Fluoresceinester und/oder mindestens ein Derivat eines Fluoresceinesters umfasst.

15. Vorrichtung gemäß einem oder mehreren der Ansprüche 10 bis 14, **dadurch gekennzeichnet, dass** die Indikatorzuführeinheit eine Kühlungeinheit zur Einstellung einer Temperatur der Indikatorlösung in einem Bereich von etwa -15°C bis etwa +6°C umfasst.

16. Vorrichtung gemäß einem oder mehreren der Ansprüche 10 bis 15, **dadurch gekennzeichnet, dass** diese weiterhin mindestens eine Biozidzuführeinheit, mindestens eine Enzymhemmerzuführeinheit und/oder mindestens eine Inaktivatorzuführeinheit (60) für oxidierende und/oder für nichtoxidierende Biozide umfasst.

## Claims

1. Method for accelerated determination of the concentration of living thermophilic bacteria in water-carrying installations, in particular in cooling water circuits, in which a bioreactor (20) is connected to a water-carrying supply line (12) with at least one first control unit (22) for an aqueous measuring solution and a discharge line (14) with at least one second control unit (24), comprising the steps of
a) charging the bioreactor (20) with the aqueous measuring solution;
b) physical and chemical deactivation of accompanying flora, biocides and/or enzymes contained in the measuring solution at an elevated temperature T1 for a period of at least about 1 min, the physical and/or chemical deactivation being carried out in such a way that the thermophilic bacteria to be determined are not deactivated;
c) reducing the temperature of the test solution to a temperature T2 to a range from about 38°C to about 45°C;
d) adding at least one indicator solution;
e) after carrying out steps a) to d), spectrometric determination of at least two measured values W1 and W2 of the measuring solution determined at different times, using an indicator solution comprising at least one indicator agent, by microbiological conversion of the at least one indicator agent; and
f) evaluating the determined at least two measured values W1 and W2 and determining a concentration of living thermophilic bacteria in the measuring solution.

2. Method according to claim 1, **characterized in that** the physical deactivation in step b) is carried out at a temperature T1 above 45°C.

3. Method according to one or more of the preceding claims, **characterized in that** the chemical deactivation is performed at least by adjusting the pH of the measuring solution to a value below about 6.5.

4. Method according to one or more of the preceding claims, **characterized in that** the deactivation of bacteria contained in the measuring solution is carried out by adding at least one broad-spectrum biocide specific for thermoplastic microorganisms which cannot be determined, the deactivation of oxidizing and/or non-oxidizing biocides present in the measuring solution is carried out by adding at least one inactivator solution and/or the deactivation of enzymes present in the measuring solution is carried out by adding at least one enzyme inhibitor.

5. Method according to one or more of the preceding claims, **characterized in that** the indicator solution in step d) is a color indicator solution, wherein a change in a color spectrum of the color indicator solution in step e) is determined at at least one wavelength.

6. Method according to one or more of the preceding claims, **characterized in that** in step e) a measured value W1 is determined in a range from about 30 min to about 90 min after completion of the adding according to step d).

7. Method according to one or more of the preceding claims, **characterized in that** in step e) a measured value W2 is determined in a range from about 120 min to about 210 min after completion of the adding according to step d).

8. Method according to one or more of the preceding claims, **characterized in that** after step e) or f) a disinfection of the measuring solution with indicator solution and a device used for carrying out the method is carried out at a temperature above about 63°C.

9. Method according to one or more of the preceding claims, **characterized in that** the determination of the concentration of living thermophilic bacteria is carried out automatically at predetermined time intervals.

10. Device (10) for carrying out a method according to one or more of the preceding claims, comprising a bioreactor (20) with a water-carrying supply line (12) with at least one first control unit (22) for an aqueous measuring solution and a discharge line (14) with at least one second control unit (24), further comprising a pH measuring device (34) which is in contact with the measuring solution in the bioreactor (20) during a measurement, a pH regulating agent unit (50) for supplying a regulating agent into the bioreactor (20), a heating means (38) for the measuring solution, at least one indicator supply unit (40) for supplying an indicator solution into the bioreactor (20), at least one spectrometer (32) and a computer-aided evaluation unit (90) for determining the concentration of living thermophilic bacteria.

11. Device according to claim 10, **characterized in that** it further comprises a stirring unit (30) for stirring the measuring solution with and without indicator solution.

12. Device according to one or more of claims 10 to 11, **characterized in that** the at least one indicator supply unit (40) comprises at least one dosing device (42) for adding the at least one indicator solution into the bioreactor (20).

13. Device according to one or more of claims 10 to 12, **characterized in that** the spectrometer (32) is a fluorometer.

14. Device according to one or more of claims 10 to 13, **characterized in that** the at least one indicator solution comprises at least one fluorescein ester and/or at least one derivative of a fluorescein ester.

15. Device according to one or more of claims 10 to 14, **characterized in that** the indicator supply unit comprises a cooling unit for adjusting a temperature of the indicator solution in a range of about -15°C to about +6°C.

16. Device according to one or more of claims 10 to 15, **characterized in that** it further comprises at least one biocide supply unit, at least one enzyme inhibitor supply unit and/or at least one inactivator supply unit (60) for oxidizing and/or for non-oxidizing biocides.

## Revendications

1. Procédé de détermination accélérée de la concentration de bactéries thermophiles vivantes dans les installations d'acheminent d'eau, en particulier dans les circuits d'eau de refroidissement, dans lequel un bioréacteur (20) avec une conduite d'alimentation en eau (12) avec au moins une première unité de contrôle (22) pour une solution de mesure aqueuse et une conduite d'évacuation (14) est connecté à au moins une deuxième unité de contrôle (24), comprenant les étapes:
a) chargement dudit bioréacteur (20) avec ladite solution de mesure aqueuse;
b) désactivation physique et chimique d'une flore accompagnatrice, des biocides et/ou des enzymes contenus dans ladite solution de mesure à une température élevée T1 sur une durée d'au moins 1 minute environ, ladite désactivation physique et/ou chimique se produit de manière à ce que lesdites bactéries thermophiles à déterminer ne soient pas désactivées ;
c) abaissement de la température de la solution de mesure à une température T2 dans une plage d'environ 38°C à environ 45°C ;
d) ajout d'au moins une solution indicatrice ;
e) après avoir exécuté les étapes a) à d), détermination spectrométrique d'au moins deux valeurs de mesure W1 et W2 de ladite solution de mesure, déterminées à des moments différents, avec une solution indicatrice comprenant au moins un agent indicateur par conversion microbiologique dudit au moins un agent indicateur; et
f) évaluation desdits au moins deux valeurs de mesure W1 et W2 déterminées et détermination d'une concentration desdites bactéries thermophiles vivantes dans ladite solution de mesure.

2. Procédé selon la revendication 1, **caractérisé en ce que** ladite désactivation physique de l'étape b) est effectuée à une température T1 supérieure à 45°C.

3. Procédé selon une ou plusieurs des revendications précédentes, **caractérisée en ce que** ladite désactivation chimique est effectuée au moins en ajustant le pH de ladite solution de mesure à une valeur inférieure à environ 6,5.

4. Procédé selon une ou plusieurs des revendications précédentes, **caractérisé en ce que** ladite désactivation desdites bactéries contenues dans ladite solution de mesure est réalisée par l'ajout d'au moins un biocide à large spectre spécifique pour des micro-organismes thermoplastiques, qui ne sont pas à déterminer, la désactivation des biocides oxydants et/ou non oxydants présents dans ladite solution de mesure est réalisée par l'ajout d'au moins une solution d'inactivation et/ou la désactivation des enzymes présentes dans ladite solution de mesure est réalisée par l'ajout d'au moins un inhibiteur d'enzyme.

5. Procédé selon une ou plusieurs des revendications précédentes, **caractérisé en ce que** ladite solution indicatrice dans l'étape d) est une solution indicatrice de couleur, dans laquelle un changement d'un spectre de couleur de ladite solution indicatrice de couleur est déterminé dans l'étape e) à au moins une longueur d'onde.

6. Procédé selon une ou plusieurs des revendications précédentes, **caractérisé en ce qu'**à l'étape e), une valeur mesurée W1 est déterminée dans une plage d'environ 30 min à environ 90 min après la fin du dosage selon l'étape d).

7. Procédé selon une ou plusieurs des revendications précédentes, **caractérisé en ce qu'**à l'étape e), une valeur mesurée W2 est déterminée dans une plage d'environ 120 min à environ 210 min après la fin du dosage selon l'étape d).

8. Procédé selon une ou plusieurs des revendications précédentes, **caractérisé en ce qu'**après l'étape e) ou f), une désinfection de ladite solution de mesure avec une solution indicatrice et un dispositif utilisé pour la réalisation du procédé est effectuée à une température supérieure à environ 63°C.

9. Procédé selon une ou plusieurs des revendications précédentes, **caractérisé en ce que** la détermination de la concentration des bactéries thermophiles vivantes est réalisé automatiquement à des intervalles de temps prédéterminés.

10. Dispositif(10) pour la réalisation d'un procédé selon une ou plusieurs des revendications précédentes, comprenant un bioréacteur (20) avec une conduite d'alimentation en eau (12) avec au moins une première unité de commande (22) pour une solution de mesure aqueuse et une conduite d'évacuation (14) avec au moins une deuxième unité de commande (24), comprenant en outre un dispositif de mesure du pH (34) qui est en contact avec ladite solution de mesure dans ledit bioréacteur (20) pendant une mesure, une unité d'agent de régulation du pH (50) pour amener un agent de régulation dans ledit bioréacteur (20), un moyen de chauffage (38) pour ladite solution de mesure, au moins une unité d'alimentation en indicateur (40) pour l'introduction d'une solution d'indicateur dans ledit bioréacteur (20), au moins un spectromètre (32) et une unité d'évaluation assistée par ordinateur (90) pour la détermination de la concentration desdites bactéries thermophiles vivantes.

11. Dispositif selon la revendication 10, **caractérisé en ce qu'**il comprend en outre une unité d'agitation (30) pour agiter ladite solution de mesure avec et sans solution indicatrice.

12. Dispositif selon une ou plusieurs des revendications 10 à 11, **caractérisé en ce que** ladite au moins une unité d'alimentation en indicateur (40) comprend au moins un dispositif de dosage (42) pour distribuer ladite au moins une solution indicatrice dans ledit bioréacteur (20).

13. Dispositif selon une ou plusieurs des revendications 10 à 12, **caractérisé en ce que** ledit spectromètre (32) est un fluorimètre.

14. Dispositif selon une ou plusieurs des revendications 10 à 13, **caractérisé en ce que** ladite au moins une solution indicatrice comprend au moins un ester de fluorescéine et/ou au moins un dérivé d'un ester de fluorescéine.

15. Dispositif selon une ou plusieurs des revendications 10 à 14, **caractérisé en ce que** ladite unité d'alimentation en indicateur comprend une unité de refroidissement pour ajuster une température de ladite solution indicatrice dans une plage d'environ -15°C à environ +6°C.

16. Dispositif selon une ou plusieurs des revendications 10 à 15, **caractérisé en ce qu'**il comprend en outre au moins une unité de délivrance de biocide, au moins une unité de délivrance d'inhibiteur d'enzyme et/ou au moins une unité de délivrance d'inactivateur (60) pour des biocides oxydants et/ou non oxydants.
